# EUROPÄISCHE PATENTANMELDUNG

(11) **EP 0 826 739 A1**
(43) Veröffentlichungstag der Anmeldung: **04.03.1998**
(21) Anmeldenummer: 97114338.3
(22) Anmeldetag: 20.08.1997
(51) Int. Cl.: C09B 1/28, C09B 1/36, C09B 1/473, C07C 221/00, C07C 231/08

(54) **Verfahren zur Herstellung substituierter Aminoanthrachinone**

(30) Priorität: 02.09.1996 DE 19635426
(71) Anmelder: BAYER AG, 51368 Leverkusen (DE)
(72) Erfinder: Kalz, Dietmar, Dr., 53819 Neunkirchen-Seelscheid (DE); Reinhardt, Karl-Heinz, 40789 Monheim (DE)

(57) **Zusammenfassung**

Ein Verfahren zur Herstellung substituierter 1-Aminoanthrachinone der Formel (I) 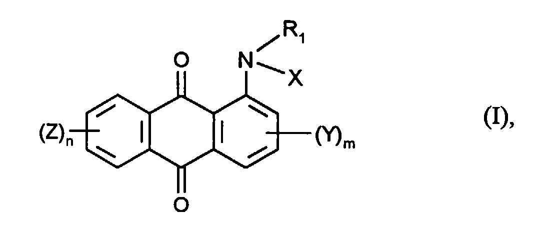 worin
- R₁: gegebenenfalls substituiertes C₁-C₆-Alkyl bedeutet und
- X, Y, Z, n und m: die in der Beschreibung angegebene Bedeutung haben
durch Umsetzung von 1-Aminoanthrachinonen der Formel (II) 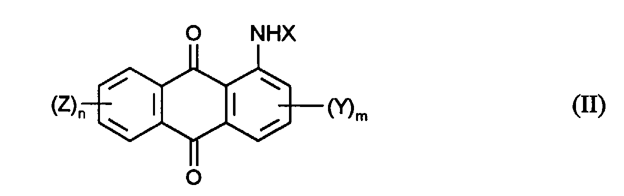 mit Alkylierungsmitteln in organischen Lösungsmitteln und in Gegenwart von Alkali, ist durch die Umsetzung in Gegenwart von Polyethern mit einem 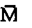 n von größer oder gleich 200 g/mol gekennzeichnet und liefert in besserer Reinheit und höherer Ausbeute substituierte 1-Aminoantrachinone, die sich besonders als Farbstoffe, Pigmente oder deren Zwischenprodukte eignen.

## Beschreibung

Die Erfindung betrifft ein Verfahren zur Herstellung substituierter Aminoanthrachinone sowie die Verwendung der so erhaltenen Aminoanthrachinone als Farbstoffe, Pigmente oder als deren Zwischenprodukte.

Substituierte 1-Aminoanthrachinone sind beispielsweise aus EP-A-368 750 bekannt und können als Farbstoffe, Pigmente oder als deren Zwischenprodukte verwendet werden. Entsprechende Farbstoffe sind beispielsweise geeignet zum Färben von Kunststoffen, Ölen oder Wachsen in der Masse.

In EP-A-368 750 werden substituierte 1-Alkyl-Aminoanthrachinone der Formel (I), wie sie unten beschrieben werden, dadurch hergestellt, daß man die entsprechend nicht alkylierten 1-Aminoanthrachinone mit Alkylierungsmitteln in organischen Lösungsmitteln und in Gegenwart von quaternären Ammoniumsalzen umsetzt. Nachteil dieser Verfahrensweise ist jedoch, daß die Raum-Zeit-Ausbeuten dieses Verfahrens noch unzureichend sind.

Aufgabe der vorliegenden Erfindung war es daher, ein Verfahren zur Herstellung substituierter Aminoanthrachinone mit verbesserten Raum-Zeit-Ausbeuten bereitzustellen.

Es wurde nun ein Verfahren zur Herstellung substituierter 1-Aminoanthrachinone der Formel (I) gefunden, 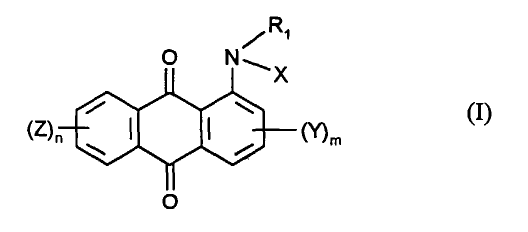 worin
- R₁: gegebenenfalls substituiertes C₁-C₆-Alkyl,
- X: Wasserstoff, -COR₂ oder -SO₂R₃ bedeutet, worin R₂ und R₃ jeweils für substituiertes oder unsubstituiertes C₁-C₄-Alkyl oder C₆-C₁₂-Aryl stehen,
- Y und Z: unabhängig voneinander jeweils gleich oder verschieden sind und Halogen, NO₂ oder C₁-C₄-Alkyl bedeuten,
- n: eine Zahl von 0 bis 4, insbesondere 0 oder 1 und
- m: eine Zahl von 0 bis 3, insbesondere 0 oder 1 bedeutet,
durch Umsetzung von 1-Aminoanthrachinonen der Formel (II) 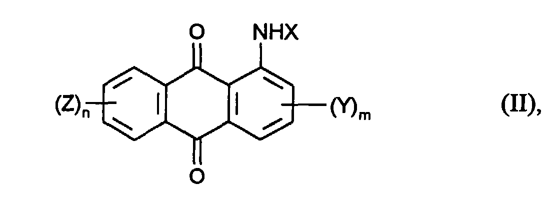 worin
- X, Y, Z, n und m: die oben angegebene Bedeutung haben,
mit Alkylierungsmitteln in organischen Lösungsmitteln und in Gegenwart von Alkali, dadurch gekennzeichnet, daß die Umsetzung in Gegenwart von Polyethern mit einem 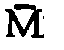 n von wenigstens 200 g/mol erfolgt.
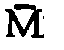 ₙ steht für mittleres als Zahlenmittel bestimmtes Molekulargewicht.

Verbindungen der Formel (II) sind ebenfalls bekannt. So können beispielsweise aus 1-Aminoanthrachinon die übrigen Verbindungen der Formel (II) durch Acylierung, Sulfonierung, Halogenierung, Nitrierung und/oder Alkylierung hergestellt werden.

Beispiele für bevorzugte Verbindungen der Formel (II) sind:
1-Aminoanthrachinon, 1-Acerylaminoanthrachinon, 4-Brom-1-acetylaminoanthrachinon, 5-Nitro-4-brom-1-acetylaminoanthrachinon, 4-Brom-5-chlor-1-acetylaminoanthrachinon, 1-Propionylaminoanthrachinon, 1-Benzoylaminoanthrachinon, 4-Brom-1-benzoylaminoanthrachinon, 1-Cyanacetylaminoanthrachinon, 1-Chloracetylaminoanthrachinon, 1-Toluylaminoanthrachinon, 1-Toluolsulfonylaminoanthrachinon, 4-Brom-1-p-toluolsulfonylaminoanthrachinon, 1-Benzolsulfonylaminoanthrachinon, 1-Methylsulfonylaminoanthrachinon und 1-Naphthalinsulfonylaminoanthrachinon.

Als Alkylierungsmittel sind beispielsweise zu nennen:
Alkylhalogenide, Dialkylsulfate oder Arylsulfonsäurealkylester.

Als geeignete Alkylhalogenide kommen beispielsweise in Frage:
Methyliodid, Ethyliodid, n-Propyliodid, i-Propyliodid, n-Butyliodid, sec.-Butyliodid, t-Butyliodid, n-Amyliodid, i-Amyliodid, n-Hexyliodid, Acryliodid, Hydroxyethyliodid, Ethoxyethyliodid und Phenoxyethyliodid sowie die entsprechenden Bromide und Chloride.

Als geeignete Dialkylsulfate sind beispielsweise zu nennen:
Dimethylsulfat und Diethylsulfat.

Als geeignete Arylsulfonsäurealkylester sind beispielsweise zu nennen:
Methyl-p-toluolsulfonat, Ethyl-p-toluolsulfonat, Methylbenzolsulfonat und Ethylbenzolsulfonat.

Das Alkylierungsmittel wird vorzugsweise in einer Menge von 1,0 bis 5,0 mol, vorzugsweise 1,5 bis 2,0 mol pro mol der Verbindung der Formel (II) eingesetzt.

Als organische Lösungsmittel sind polare Lösungsmittel bevorzugt. Besonders bevorzugt sind polare Lösungsmittel mit einem elektrischen Dipolmoment von größer als 2,5 D. Solche sind beispielsweise Nitrobenzol, Sulfolan, Tetrahydrofuran, Dimethylformamid, N-Alkylpyrrolidone wie N-Methylprrolidon, Ketone wie Methylisopropylketon oder 5-Methyl-3-heptanon.

Das verwendete Lösungsmittel ist von dem eingesetzten Polyether verschieden.

Die für das erfindungsgemäße Verfahren eingesetzten Alkali-Verbindungen sind beispielsweise NaOH, KOH, Alkalicarbonate wie Na₂CO₃ oder K₂CO₃. Bevorzugt sind NaOH und KOH. Sie werden vorzugsweise in einer Menge von 1 bis 5 mol, insbesondere 1,5 bis 2 mol pro mol der Verbindung der Formel (II) eingesetzt.

Als bevorzugte Polyether mit einem 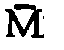 n von wenigstens 200 g/mol sind beispielsweise Kronenether, insbesondere solche aus Alkylenoxideinheiten, insbesondere Ethylenoxideinheiten, wie 15-Krone-5-, 12-Krone-4- oder 18-Krone-6-Ether und/oder alkoxylierte Alkohole und Carbonsäuren zu nennen.

Besonders bevorzugte Polyether sind Polyalkylenglykole, insbesondere Polyethylenglykole mit einem als Zahlenmittel bestimmten mittleren Molekulargewicht von 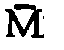 n = 200 bis 5 000 g/mol.

Ebenfalls bevorzugt sind Polyalkylenglykolmonoalkylether auf Basis der obengenannten Polyalkylenglykole wie beispielsweise monomethylierte, -ethylierte oder -butylierte Polyalkylenglykole.

Ebenfalls bevorzugte Polyether sind polyalkoxylierte Fettalkohole, insbesondere ethoxylierte Fettalkohole. Als Fettalkohole sind dabei vorzugsweise die gesättigten oder ungesättigten primären C₆-C₂₂-Alkohole wie beispielsweise Oleylalkohol und Laurylalkohol zu nennen, die mit vorzugsweise 2 bis 100 Einheiten Ethylenoxid alkoxyliert sind.

Aber auch Phenole wie Nonylphenol führen nach einer Polyalkoxylierung, insbesondere Ethoxylierung mit 2 bis 100 Ethylenoxideinheiten zu erfindungsgemäß vorteilhaft einsetzbaren Polyethern.

Als zu alkoxylierende, insbesondere zu ethoxylierende Carbonsäuren sind beispielsweise gesättigte und ungesättigte Carbonsäuren mit 8 bis 20 -C-Atomen, wie Stearinsäure oder Ölsäure zu nennen.

Auch die auf Carbonsäuren basierenden Polyether werden vorzugsweise durch Ethoxylierung mit 2 bis 100 Ethylenoxideinheiten pro Carbonsäure erhalten.

Besonders bevorzugte Polyether sind: Polyethylenglykol mit einem als Zahlenmittel bestimmten mittleren Molekulargewicht 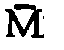 n = 400 bis = 1550 g/mol, Umsetzungsprodukte von Oleylalkohol mit 10 bis 19 Mol-Äquivalenten Ethylenoxid (EO), Laurylalkohol mit 7 Mol-Äquivalenten EO, Ölsäure mit 6 Mol-Äquivalenten EO, Nonylphenol mit 10,40 oder 100 Mol-Äquivalenten EO.

Das erfindungsgemäße Verfahren wird vorzugsweise bei einer Temperatur von 0 bis 50°C, insbesondere bei 0 bis 20°C, besonders bevorzugt bei 10 bis 15°C durchgeführt.

Die Menge des verwendeten Polyethers beträgt vorzugsweise 3 bis 20 Gew.-%, insbesondere 5 bis 10 Gew.-%, bezogen auf das Aminoanthrachinon der Formel (II).

Die Aufarbeitung erfolgt im allgemeinen so, daß zur Reaktionsmischung nach beendeter Reaktion ausreichend Wasser zugefügt wird, um überschüssiges Alkylierungsmittel zu zersetzen. Das Reaktionsprodukt des erfindungsgemäßen Verfahrens wird vorzugsweise durch Filtration isoliert, mit Wasser gewaschen und dann getrocknet.

Die alkylierten Aminoanthrachinone der Formel (I) fallen mit so hoher Reinheit an, daß sie ohne weitere Reinigung ihrer Verwendung zugeführt werden können. Insbesondere genügen die so erhaltenen Produkte den hohen Reinheitsanforderungen als Farbstoffe zum Färben von Kunststoffen in der Masse.

Unter Massefärben werden hierbei insbesondere Verfahren verstanden, bei denen der Farbstoff in die geschmolzene Kunststoffmasse eingearbeitet wird, z.B. unter Zuhilfenahme eines Extruders, oder bei denen der Farbstoff bereits Ausgangskomponenten zur Herstellung des Kunststoffes, z.B. Monomeren vor der Polymerisation, zugesetzt wird.

Besonders bevorzugte Kunststoffe sind Thermoplaste, beispielweise Vinylpolymere, Polyester, Polyamide oder Polycarbonate.

Geeignete Vinylpolymere sind Polystyrol, Styrol-Acrylnitril-Copolymere, Styrol-Butadien-Copolymere, Styrol-Butadien-Acrylnitril-Terpolymere, Polymethacrylat, Polyvinylchlorid u.a.

Weiterhin geeignete Polyester sind: Polyethylenterephthalate, Polycarbonate und Celluloseester.

Bevorzugt sind Polystyrol, Styrol-Mischpolymere, Polycarbonate und Polymethacrylat. Besonders bevorzugt ist Polystyrol.

Die erwähnten hochmolekularen Verbindungen können einzeln oder in Gemischen, als plastische Massen oder Schmelzen vorliegen.

Die erfindungsgemäßen Farbstoffe werden in feinverteilter Form zur Anwendung gebracht, wobei Dispergiermittel mitverwendet werden können aber nicht müssen.

Werden die nach dem erfindungsgemäßen Verfahren erhaltenen Verbindungen der Formel (I) nach der Polymerisation eingesetzt, so werden sie vorzugsweise mit dem Kunststoffgranulat trocken vermischt oder vermahlen und dieses Gemisch z.B. auf Mischwalzen oder in Schnecken plastifiziert und homogenisiert. Man kann die Farbstoffe aber auch der schmelzflüssigen Masse zugeben und diese durch Rühren homogen verteilen. Das derart vorgefärbte Material wird dann wie üblich z.B. durch Verspinnen zu Borsten, Fäden usw. oder durch Extrusion oder im Spritzguß-Verfahren zu Formteilen weiterverarbeitet.

Die Verbindungen der Formel (I) werden vorzugsweise zum Färben der genannten Polymeren in Mengen von 0,0001 bis 1 Gew..-%, insbesondere 0,01 bis 0,5 Gew.-%, bezogen auf die Polymermenge, eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

### Beispiele

### Beispiel 1

250 ml N-Methylpyrrolidon wurden unter Rühren mit 34,2 g (0,15 Mol) 1-Aminoanthrachinon und 3,0 g Polyethylenglykol ( 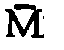 n 1 550 g/mol) versetzt. Man fügte 24,8 g Kaliumhydroxid, 85 %ig (Rest Wasser) zu und kühlte auf 5°C ab. Dann wurden bei 5 bis 15°C 37,5 g (0,3 Mol) Dimethylsulfat in ca. 10 Min. zugegeben. Gegen Ende der Zugabe kristallisierte bereits das 1-Methylaminoanthrachinon aus. Man gab 250 ml Wasser zu, hielt 1 Stunde auf 60°C, filtrierte dann den Farbstoff ab, wusch mit 250 ml Wasser und trocknete im Vakuum.
- Ausbeute:: 34,5 g = 95 % d. Th.
- Reingehalt:: 98.0 % (HPLC) 1-Methylaminoanthrachinon

### Beispiel 2

250 ml Dimethylformamid wurden unter Rühren in 34,2 g (0,15 Mol) 1-Aminoanthrachinon und 3,0 g Polyether aus Oleylalkohol und 50 mol Ethylenoxid versetzt. Darauf wurden 24,8 g Kaliumhydroxid, 85 %ig (Rest Wasser) zugegeben und man kühlte auf 10°C. Dann gab man während ca. 10 Min. bei 10 bis 15°C, 37,5 g (0,3 Mol) Dimethylsulfat zu, ließ noch 15 Min. rühren bis sich der Ansatz auf ca. 20°C erwärmt hatte und fügte danach 250 ml Wasser zu. Man erwärmte auf 60°C, hält 1 Stunde bei dieser Temperatur und filtrierte den Farbstoff ab. Anschließend wurde mit 250 ml Wasser gewaschen und im Vakuum getrocknet.
- Ausbeute:: 35,5 g = 97,7 % d. Th.
- Reingehalt:: 99,0 % (HPLC)

### Beispiel 3

225 ml 5-Methyl-3-heptanon wurden mit 16,5 g Kaliumhydroxid, 85 %ig, 22,4 g (0,1 Mol) 1-Aminoanthrachinon und 3,0 g Polyether aus Oleylalkohol und 19 mol Ethylenoxid versetzt, auf 5°C abgekühlt und dann bei 5 bis 10°C mit 25,0 g (0,2 Mol) Dimethylsulfat verrührt. Dann wurde noch 15 Min. bei Raumtemperatur verrührt und schließlich mit 175 ml Methanol verdünnt. Der auskristallisierte Farbstoff wurde abfiltriert, mit 200 ml Methanol und danach mit 200 ml heißem Wasser gewaschen. Das 1-Methyl-aminoanthrachinon wurde bei 70°C im Vakuum getrocknet.
- Ausbeute:: 22,6 g = 95 % d. Th.
- Reingehalt:: 98,9 % (HPLC) 1-Methylaminoanthrachinon

### Beispiel 4

250 ml N-Methylpyrrolidon wurden unter Rühren mit 26,5 g (0,1 Mol) 1-Acetylaminoanthrachinon, 16,5 g Kaliumhydroxid 85 %ig, 3,0 g Polyether aus Oleylalkohol und 50 mol Ethylenoxid versetzt. Danach wurden bei 10 bis 15°C 25,0 g (0,2 Mol) Dimethylsulfat zugegeben und 30 Min. bei max. 20°C verrührt. Darauf wurden 250 ml Methanol zugegeben und das N-Acetylamino-1-methylaminoanthrachinon abfiltriert. Nach dem Waschen mit 200 ml Methanol wurde mit 200 ml Wasser das Produkt gewaschen und im Vakuum getrocknet.
- Ausbeute:: 26,5 g = 95 % d. Th.
- Reingehalt:: 96 % (HPLC)

## Patentansprüche

1. Verfahren zur Herstellung substituierter 1-Amino-Anthrachinone der Formel (I) 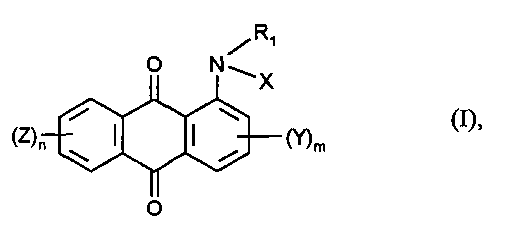 worin
R₁ gegebenenfalls substituiertes C₁-C₆-Alkyl,
X Wasserstoff, -COR₂ oder -SO₂R₃ bedeutet, worin R₂ und R₃ jeweils für substituiertes oder unsubstituiertes C₁-C₄-Alkyl oder C₆-C₁₂-Aryl stehen und
Y und Z unabhängig voneinander, jeweils gleich oder verschieden sind und Halogen, NO₂ oder C₁-C₄-Alkyl bedeuten,
n eine Zahl von 0 bis 4, insbesondere 0 oder 1 und
m eine Zahl von 0 bis 3, insbesondere 0 oder 1 bedeuten,
durch Umsetzung von 1-Aminoanthrachinonen der Formel (II) 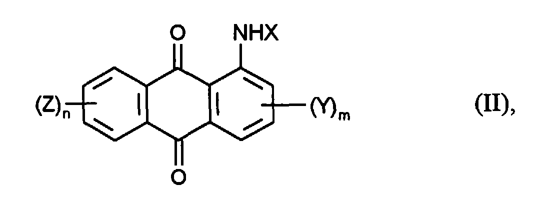 worin X, Y, Z, n und m die oben angegebene Bedeutung haben, mit Alkylierungsmitteln in organischen Lösungsmitteln und in Gegenwart von Alkali, dadurch gekennzeichnet, daß die Umsetzung in Gegenwart von Polyethern mit einem 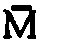 n von wenigstens 200 g/mol erfolgt.

2. Verfahren gemäß Anspruch 1, dadurch gekennzeichnet, daß als Verbindung der Formel (II) 1-Aminoanthrachinon, 1-Acetylaminoanthrachinon, 4-Brom-1-acetylaminoanthrachinon, 5-Nitro-4-brom-1-acetylaminoanthrachinon, 4-Brom-5-chlor-1-acetylaminoanthrachinon, 1-Propionylaminoanthrachinon, 1-Benzoylaminoanthrachinon, 4-Brom-1-benzoylaminoanthrachinon, 1-Cyanacetylaminoanthrachinon, 1-Chloracetylaminoanthrachinon, 1-Toluylaminoanthrachinon, 1-Toluolsulfonylaminoanthrachinon, 4-Brom-1-p-toluolsulfonylaminoanthrachinon, 1-Benzolsulfonylaminoanthrachinon, 1-Methylsulfonylaminoanthrachinon oder 1-Naphthalinsulfonylaminoanthrachinon eingesetzt wird.

3. Verfahren gemäß Anspruch 1, dadurch gekennzeichnet, daß als Alkylierungsmittel Alkylhalogenide, Dialkylsulfate oder Alylsulfonsäurealkylester verwendet werden.

4. Verfahren gemäß Anspruch 1, dadurch gekennzeichnet, daß als Alkylierungsmittel Methyliodid, Ethyliodid, n-Propyliodid, i-Propyliodid, n-Butyliodid, sec.-Butyliodid, t-Butyliodid, n-Amyliodid, i-Amyliodid, n-Hexyliodid, Acryliodid, Hydroxyethyliodid, Ethoxyethyliodid oder Phenoxyethyliodid oder die entsprechenden Bromide oder Chloride, Dimethylsulfat oder Diethylsulfat oder Methyl-p-Toluolsulfonat, Ethyl-p-toluolsulfonat, Methylbenzolsulfonat oder Ethylbenzolsulfonat eingesetzt wird.

5. Verfahren gemäß Anspruch 1, dadurch gekennzeichnet, daß das organische Lösungsmittel polar ist, insbesondere ein elektrisches Dipolmoment von größer als 2,5 D besitzt.

6. Verfahren gemäß Anspruch 1, dadurch gekennzeichnet, daß als organisches Lösungsmittel Nitrobenzol, Sulfolan, Tetrahydrofuran, Dimethylformamid, ein N-Alkylpyrrolidon oder ein Keton eingesetzt wird.

7. Verfahren gemäß Anspruch 1, dadurch gekennzeichnet, daß als Polyether Kronenether, insbesondere solche aus Alkylenoxideinheiten, insbesondere Ethylenoxideinheiten und/oder alkoxylierte Alkohole und Carbonsäuren eingesetzt werden.

8. Verfahren gemäß Anspruch 1, dadurch gekennzeichnet, daß als Polyether Polyalkylenglykole, Polyalkylenglykolmonoalkylether, polyalkoxylierte Fettalkohole, polyalkoxylierte Phenole oder polyalkoxylierte Carbonsäuren eingesetzt werden.

9. Verfahren gemäß Anspruch 1, dadurch gekennzeichnet, daß die Umsetzung bei einer Temperatur von 0 bis 50°C, insbesondere von 0 bis 20°C, besonders bevorzugt von 10 bis 15°C durchgeführt wird.

10. Verwendung der nach Anspruch 1 erhaltenen substituierten 1-Aminoanthrachinone als Farbstoff, Pigment oder als deren Zwischenprodukt.
